(19) Europäisches Patentamt
European Patent Office
Office européen des brevets

(11) Veröffentlichungsnummer: **0 180 834**
**B1**

(12) # EUROPÄISCHE PATENTSCHRIFT

(45) Veröffentlichungstag der Patentschrift: **30.08.89**

(51) Int. Cl.[4]: **C 07 D471/04**, A 61 K 31/505 // (C07D471/04, 239:00, 221:00)

(21) Anmeldenummer: **85113270.4**

(22) Anmeldetag: **18.10.85**

(54) **4-Alkoxy-pyrido[2,3-d]pyrimidin-Derivate, Verfahren zu deren Herstellung und diese enthaltende Arzneimittel.**

(30) Priorität: **19.10.84 DE 3438351**

(43) Veröffentlichungstag der Anmeldung: **14.05.86 Patentblatt 86/20**

(45) Bekanntmachung des Hinweises auf die Patenterteilung: **30.08.89 Patentblatt 89/35**

(84) Benannte Vertragsstaaten: **AT BE CH DE FR GB IT LI LU NL SE**

(56) Entgegenhaltungen:
**BE-A- 525 394**
**US-A- 3 673 184**
**ARZNEIMITTEL FORSCHUNG, Band 31(II), Nr. 8, 1981, Seiten 1173-1177, Aulendorf, DE; VON H. MEYER et al.: "2-Aminodihydropyridine Struktur und antihypertensive Wirksamkeit"**
**Die Akte enthält technische Angaben, die nach dem Eingang der Anmeldung eingereicht wurden und die nicht in dieser Patentschrift enthalten sind.**

(73) Patentinhaber: **GÖDECKE AKTIENGESELLSCHAFT**
**Salzufer 16**
**D-1000 Berlin 10 (DE)**

(72) Erfinder: **Kleinschroth, Jürgen, Dr.**
**Freiburger Strasse 13**
**D-7809 Denzlingen (DE)**
Erfinder: **Satzinger, Gerhard, Dr.**
**Im Mattenbühl 7**
**D-7809 Denzlingen (DE)**
Erfinder: **Mannhardt, Karl, Dr.**
**Pfauenstrasse 14**
**D-7807 Elzach-Oberprechtal (DE)**
Erfinder: **Hartenstein, Johannes, Dr.**
**Fohrenbühl 23**
**D-7801 Stegen-Wittental (DE)**
Erfinder: **Osswald, Hartmut, Prof. Dr.**
**Kiefernweg 1**
**D-7808 Waldkirch 2 (DE)**
Erfinder: **Weinheimer, Günter, Dr.**
**Sachsenstrasse 4**
**D-7809 Denzlingen (DE)**
Erfinder: **Fritschi, Edgar, Dr.**
**Am Scheuerwald 2**
**D-7811 St. Peter (DE)**

**EP 0 180 834 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue 4-Alkoxy-pyrido [2,3-d] pyrimidin- Derivate der allgemeinen Formel I

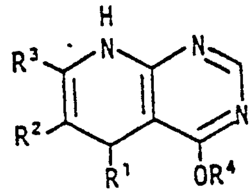

(I)

in welcher

$R^1$ einen unsubstituierten oder einen vorzugsweise in 2- oder 3- Position durch Halogen-, Nitro-, Methyl-, Methoxy-, Difluormethoxy-, Trifluormethoxy-, Dimethyl- oder Diethylamino-, Methylthio- oder Trifluormethyl-substituierten oder einen vorzugsweise in 2,3- durch Methoxy- oder Methylendioxy- oder in 2,3- oder 2,6- Position durch Halogenatome, die gleich oder verschieden sein können, disubstituierten Phenylrest,

$R^2$ eine Nitrilgruppe, eine Carboxylgruppe oder einen Alkoxycarbonylrest der allgemeinen Formel II

$$—CO_2R^5 \quad (II)$$

in welcher $R^5$ einen Methyl-, Ethyl-, n-Propyl-, Isopropyl-, n-Butyl- oder Isobutylrest sowie eine Niederalkoxyalkylgruppe darstellt,

$R^3$ einen Methyl- oder Ethylrest oder eine Aminogruppe,

$R^4$ einen Methyl-, Ethyl-, n-Propyl- oder Isopropylrest bedeutet.

sowie gegebenfalls deren pharmakologisch unbedenkliche Salze.

Die Erfindung betrifft weiterhin ein Verfahren zur Herstellung von 4-Alkoxy-pyrido [2,3-d] pyrimidin-Derivaten der allgemeinen Formel I, das dadurch gekennzeichnet ist, daß man 4-Oxo-pyrido- [2,3-d] pyrimidin-Derivate der allgemeinen Formel III

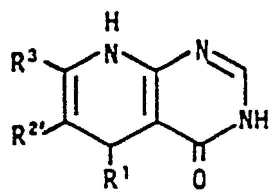

(III)

in der $R^1$ und $R^3$ die oben angegebene Bedeutung haben und $R^{2'}$ eine Nitrilgruppe oder einen Alkoxycarbonylrest mit bis zu 6 Kohlenstoffatomen bedeutet, in an sich bekannter Weise O-alkyliert.

Die als Ausgangsprodukte dienenden Verbindungen der allgemeinen Formel III werden hergestellt, indem man entweder

a) ein Dihydropyridin der allgemeinen Formel IV

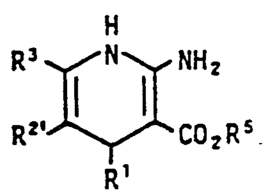

(IV)

in welcher $R^1$, $R^{2'}$, $R^3$ und $R^5$ die oben angegebene Bedeutung haben in Gegenwart einer Base mit s-Triazin umsetzt oder

b) eine Verbindung der allgemeinen Formel V

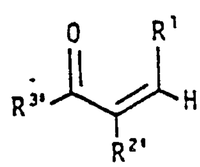

(V)

in welcher $R^1$ und $R^{2'}$ die oben angegebene Bedeutung haben und $R^{3'}$ eine geradkettige oder verzweigte Alkylgruppe mit bis zu 4 Kohlenstoffatomen bedeutet, durch Erhitzen in einem polaren Lösungsmittel mit

6-Amino-4-hydroxypyrimidin kondensiert.

Verbindungen der allgemeinen Formel I, bei denen $R^2$ für eine Carboxylgruppe steht, werden hergestellt, indem man Verbindungen der allgemeinen Formel I, in denen $R^2$ für einen zur Esterspaltung geeigneten Alkoxycarbonylrest steht, in an sich bekannter Weise bevorzugt in saurem Medium hydrolysiert..

Die Verbindungen der allgemeinen Formel IV (vgl. z. B. Liebig's Ann. Chem. 1977, S. 1895 oder Arzneim. Forsch. 31 (II), 8 (1981), S. 1173) und V (vgl. z. B. Arch. Pharm. 317 (1984), S. 709) sind literaturbekannt oder können in analoger Weise hergestellt werden.

Aus der BE-A-525 394 sind aromatische Pyrimidin-Derivate bekannt geworden. Diese weisen jedoch im Pyridinring keine polaren Substituenten auf und sind im Pyrimidinring nicht durch eine Alkoxygruppe substituiert. Sie weisen daher nicht die erfindungsgemäße Wirksamkeit auf und werden lediglich als Zwischenprodukte bzw. bakterizide Mittel eingesetzt.

Zur Durchführung der Reaktion a) wird das Dihydropyridinderivat mit s-Triazin in einem inerten organischen Lösungsmittel in Gegenwart starker Basen wie z. B. Alkalialkoholaten oder Natriumhydrid in einem inerten organischen Lösungsmittel auf Temperaturen von 50-160 °C, vorzugsweise jedoch 100-150 °C, erhitzt. Als Solventien eignen sich hier vor allem polare Lösungsmittel wie Dimethylsulfoxid, Dimethylformamid oder Ethylenglykoldimethylether.

Bei dieser Reaktion entstehen neben den jeweils als Hauptprodukte isolierten Verbindungen der allgemeinen Formel III auch Verbindungen gemäß Patentanmeldung P 33 27 650 die durch Chromatographie abgetrennt werden.

Die Reaktion b) wird durch Erhitzen beider Komponenten in saurem Medium, vorzugsweise in siedendem Eisessig, durchgeführt.

Die Herstellung der 4-Alkoxy-pyrido [2,3-d] pyrimidin-Derivate der allgemeinen Formel I erfolgt erfindungsgemäß nach üblichen, für die O-Alkylierung von Lactamen in der Literatur beschriebenen Verfahren (vgl. Adv. Heterocyclic Chem. 12 (1970), 185-212). Geeignete Alkylierungsmittel sind Alkylhalogenide und Alkylsulfonate, Dialkylsulfate und Trialkyloxoniumsalze.

Zur Reaktion mit Alkylhalogeniden werden die Verbindungen der allgemeinen Formel III in Form ihrer Metallsalze, vorzugsweise ihrer Alkali-oder Silbersalze eingesetzt, die entweder separat hergestellt oder in situ mit Hilfe geeigneter Basen wie Metallhydriden, -carbonaten oder -alkoxiden in einem aprotischen Lösungsmittel erzeugt werden. Als geeignete Lösungsmittel kommen, in Abhängikeit von jeweiligen Alkylierungsmittel, nahezu alle inerten organischen Lösungsmittel in Frage wie offenkettige, cyclische oder auch aromatische Kohlenwasserstoffe, z. B. n-Pentan, n-Hexan, Cyclohexan, Benzol oder Toluol, halogenierte Kohlenwasserstoffe wie Dichlormethan und 1,2-Dichlorethan, Ether wie z. B. Diethylether und 1,2-Dimethoxyethan, sowie dipolare aprotische Lösungsmittel wie Dimethylformamid, Hexamethylphosphorsäuretriamid und Dimethylsulfoxid. Der Temperaturbereich kann je nach verwendetem Lösungsmittel zwischen — 20 °C und dem Siedepunkt des betreffenden Lösungsmittels variiert werden.

Aufgrund des ambidenten Charakters des Lactamanions erhält man bei der Alkylierung je nach Reaktionsbedingungen und verwendetem Alkylierungsmittel vielfach Gemische aus O- und N-Alkylierungsprodukten (J. Org. Chem. 32 (1967), 4040 ff).

Die Trennung der erhaltenen Produktgemische kann durch chromatographische Methoden und/oder Kristallisation erfolgen.

Die 4-Alkoxy-pyrido [2,3-d] pyrimidin-Derivate der allgemeinen Formel I werden bevorzugt durch Umsetzung der 4-Oxo-pyrido- [2,3-d] pyrimidine der allgemeinen Formel III mit Trialkyloxoniumsalzen, insbesondere Trimethyloxoniumtetrafluoroborat, in einem aprotischen Lösungsmittel erhalten. Die Herstellung der O-Isopropylverbindungen erfolgt dagegen vorteilhaft durch Alkylierung der Alkalimetallsalze mit Isopropylhalogeniden.

Da die erfindungsgemäßen Verbindungen der allgemeinen Formel I am C-5 ein chirales Zentrum aufweisen, können sie entweder als racemische Gemische oder in Form der Enantiomeren vorliegen.

Die Salze werden in üblicher Weise durch Neutralisation der Basen mit entsprechenden anorganischen oder organischen Säuren erhalten. Als Säuren kommen z. B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure oder Bernsteinsäure in Frage.

Die Verbindung der allgemeinen Formel I weisen wertvolle pharmakologische Eigenschaften auf. Insbesondere zeigen sie calciumantagonistische Wirkungen wie z. B. Aufhebung der calciuminduzierten Kontraktion des glatten Muskels bei Kaliumdepolarisation.

Aufgrund ihrer gefäßspasmolytischen Wirkungen sind sie vor allem bei cerebralen, cardialen und peripheren Gefäßerkrankungen wie myokardialer Ischämie, bei cerebralem Infarkt, pulmonalen Thrombosen und bei Arteriosklerose und anderen stenotischen Erscheinungen indiziert. Die 4-Alkoxy-pyrido [2,3-d] pyrimidin-Derivate der vorliegenden Erfindung sind daher wertvolle Mittel für die Bekämpfung der Herz-Kreislauf-Mortalität. Ein weiterer Gegenstand der vorliegenden Erfindung ist daher die Verwendung von 4-Alkoxy-pyrido [2,3-d] pyrimidin-Derivaten der allgemeinen Formel I zur Herstellung von Arzneimitteln für die Bekämpfung von Gefäßerkrankungen.

Die erfindungsgemäßen Verbindungen der allgemeinen Formel I können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder

Puffer enthält.

Derartige Zusätze sind z. B. Tartrat- und Citrat-Puffer, Ethanol, Komplexbildner (wie Äthylendiamintetraessigsäure und deren nichttoxische Salze) sowie hochmolekulare Polymere (wie flüssiges Polyäthylenoxid) zur Viskositätsregulierung. Feste Trägerstoffe sind z. B. Stärke, Lactose, Mannit, Methylcellulose, Talkum, hochdisperse Kieselsäure, höhermolekulare Fettsäure (wie Stearinsäure), Gelatine, Agar-Agar, Calciumphosphat, Magnesiumstearat, tierische und pflanzliche Fette, feste hochmolekulare Polymere (wie Polyäthylenglykol); für orale Applikation geeignete Zubereitungen können gewünschtenfalls zusätzliche Geschmacks- und/oder Süßstoffe enthalten.

Die enteral verabreichten Einzeldosen liegen im Bereich von etwa 5 bis 250 mg, vorzugsweise 10 - 100 mg. Parenteral werden etwa 1 bis 20 mg gegeben.

Die folgenden Beispiele sollen die Erfindung näher erläutern :

## Beispiel 1

(±)-5,8-Dihydro-4-isopropoxy-7-methyl-5-phenyl-pyrido [2,3-d]- pyrimidin-6-carbonsäureethylester

Zu einer gerührten Suspension von 1,0 g (34 mMol) Natriumhydrid (80%ig in Paraffinöl) in 60 ml trockenem Dimethylformamid wird eine Lösung von 6,5 g (21 mMol) (±)-3, 4, 5, 8-Tetrahydro-7-methyl-4-oxo-5-phenyl-pyrido [2,3-d] pyrimidin-6-carbonsäureethylester in 60 ml Dimethylformamid getropft. Bei Nachlassen der Gasentwicklung wird noch 30 Minuten bei Raumtemperatur gerührt; anschließend werden 5,1 g (30 mMol) Isopropyljodid in 15 ml Dimethylformamid zugetropft.

Es wird 20 Stunden bei Raumtemperatur gerührt, das Lösungsmittel im Vakuum abgezogen und der Rückstand mit 100 ml Wasser verrührt. Die gebildeten Kristalle werden abfiltriert, getrocknet, in Essigsäureethylester gelöst und an Kieselgel mit Toluol/Essigsäureethylester 1 :1 chromatographiert.

Die Fraktion mit dem $R_f$ 0,5 wird isoliert und aus Diisopropylether/Ethanol umkristallisiert. Man erhält (±)-5,8-Dihydro-4-isopropoxy-7-methyl-5-phenyl-pyrido [2,3-d] pyrimidin-6-carbonsäureethylester in Form farbloser Kristalle vom Schmp. 201-202 °C.

Der als Ausgangsmaterial verwendete (±)-3, 4, 5, 8-Tetrahydro-7-methyl-4-oxo-5-phenyl-pyrido [2,3-d] pyrimidin-6-carbonsäure-ethylester wird wie folgt hergestellt :

(±)-3, 4, 5, 8-Tetrahydro-7-methyl-4-oxo-5-phenyl-pyrido [2,3-d] pyrimidin-6-carbonsäureethylester (Verfahren a)

Zu einer gerührten Suspension von 4,5 g (150 mMol) Natriumhydrid (80 %ig in Paraffinöl) in 75 ml trockenem Dimethylformamid wird unter Stickstoffatmosphäre eine Lösung von 40,6 g (123 mMol) (±)-2-Amino-1,4-dihydro-6-methyl-4-phenylpyridin-3,5-dicarbonsäurediethylester in 200 ml Dimethylformamid getropft. Bei Nachlassen der Gasentwicklung wird noch 30 Minuten bei Raumtemperatur gerührt; anschließend werden 10,0 g (123 mMol) s-Triazin in 250 ml Dimethylformamid zugetropft. Die Reaktionsmischung wird 16 Stunden auf 110 °C erhitzt und nach dem Abkühlen im Vakuum eingeengt. Der dunkle Rückstand wird an Kieselgel mit Dichlormethan/Methanol 95 :5 chromatographiert. Die Fraktion mit dem $R_f$ 0,5 wird isoliert, mit Aceton zum Sieden erhitzt und die nach dem Abkühlen ausgefallenen Kristalle zur weiteren Reinigung aus Ethanol umkristallisiert.

Man erhält (±)-3,4,5,8-Tetrahydro-7-methyl-4-oxo-5-phenyl-pyrido-[2,3-d]pyrimidin-6-carbonsäureethylester in Form beiger Kristalle vom Schmp. 303-305 °C (Z).

In analoger Weise werden die folgenden Verbindungen erhalten :

(±)-5-(2-Fluorphenyl)-5,8-dihydro-4-isopropoxy-7-methyl-pyrido-[2,3-d]pyrimidin-6-carbonsäureethylester (1.a)

Schmp. 186-187 °C aus Diisopropylether/Ethanol

(±)-5,8-Dihydro-4-isopropoxy-7-methyl-5-(3-nitrophenyl)-pyrido-[2,3-d]pyrimidin-6-carbonsäure-(2-methoxyethyl)ester (1.b)

Schmp. 170-172 °C aus Diisopropylether/Isopropanol

(±)-5,8-Dihydro-4-isopropoxy-7-methyl-5-(2-trifluormethylphenyl)-pyrido[2,3-d]pyrimidin-6-carbonsäuremethylester (1.c)

Schmp. 214-215 °C aus Diisopropylether/Methanol

(±)-5,8-Dihydro-4-isopropoxy-7-methyl-5-(3-nitrophenyl)-pyrido-[2,3-d]pyrimidin-6-carbonsäureisopropylester (1.d)

Schmp. 181-182 °C aus Diisopropylether/Isopropanol

(±)-7-Amino-5,8-dihydro-4-isopropoxy-5-(2-methoxyphenyl)-pyrido-[2,3-d]pyrimidin-6-carbonsäureethylester (1.e).

Schmp. 222-223 °C aus Diisopropylether/Essigsäureethylester

(±)-5,8-Dihydro-4-isopropoxy-7-methyl-5-(2-trifluormethyl-phenyl)-pyrido[2,3-d]pyrimidin-6-carbonsäureethylester (1.f)

Schmp. 155 °C aus Diisopropylether

(±)-5,8-Dihydro-4-isopropoxy-7-methyl-5-(3-nitrophenyl)-pyrido-[2,3-d]pyrimidin-6-carbonsäuremethylester (1.g)

Schmp. 218-219 °C aus Diisopropylether/Methanol

Beispiel 2

(±)-5,8-Dihydro-4-methoxy-7-methyl-5-(3-nitrophenyl)-pyrido[2,3-d]pyrimidin-6-carbonsäureisopropylester

3,7 g (10 mMol) (±)-3,4,5,8-Tetrahydro-7-methyl-5-(3-nitrophenyl)-4-oxo-pyrido[2,3-d]pyrimidin-6-carbonsäureisopropylester und 3,0 g (20 mMol) Trimethyloxonium-tetrafluoroborat werden in 150 ml 1,2-Dichlorethan unter Stickstoffatmosphäre 3 Stunden bei Raumtemperatur gerührt. Es wird zweimal mit 50 ml gesättigter Natriumhydrogencarbonatlösung ausgeschüttelt, die organische Phase abgetrennt, über Natriumsulfat getrocknet und im Vakuum eingeengt. Durch zweifache Umkristallisation des Rückstands aus Diisopropylether/Isopropanol erhält man das Produkt in Form farbloser Kristalle vom Schmp. 212-213 °C.

Die folgenden Vergleichsversuche an isolierten Muskeln veranschaulichen die pharmakologische Wirksamkeit der Vergindungen gemäß der allgemeinen Formel I :

Isolierte glatte Muskeln (Tabelle 1)

von Kaninchen (Gefäßringe. von A. basilaris und A. coronaria) werden im Organbad so eingespannt, daß isometrische Kontraktionen gemessen werden können. Die Kontraktion wird durch eine Kaliumdepolarisation in Tyrodelösung ausgelöst. Diese Versuchsanordnung ist ein bekanntes Standardmodell zur Erkennung von Substanzen, die die in der Kaliumdepolarisation geöffneten Calciumkanäle blockieren (Fleckenstein, Calcium Antagonism in Heart § Smooth Muscle, J. Wiley § Sons, 1983).

Tabelle 1

Konzentrationen ($IC_{50}$, mol/1) von Verbindungen, die eine halbmaximale Hemmung der $K^+$-Depolarisationskontraktur der Gefäßringe im Organbad bewirken. A. bas. = Arteria basilaris, A. cor. = Arteria coronaria, des Kaninchens ; mittlerer Durchmesser 0,5-1,0 mm.

| Beisp.Nr. | A. basilaris | A. coronaria |
|---|---|---|
| 1b | $3.9 \times 10^{-8}$ | $3.4 \times 10^{-8}$ |
| 1c | $4 \times 10^{-8}$ | $4.5 \times 10^{-8}$ |
| 1d | $3.3 \times 10^{-8}$ | $1.6 \times 10^{-7}$ |
| 1e | $2.8 \times 10^{-6}$ | $7.3 \times 10^{-8}$ |
| 1f | $3.8 \times 10^{-8}$ | $3.4 \times 10^{-8}$ |
| 1g | $2.8 \times 10^{-8}$ | $2.3 \times 10^{-7}$ |
| 2 | $2.5 \times 10^{-9}$ | $1.4 \times 10^{-7}$ |

**Patentansprüche** (für die Vertragsstaaten : BE, CH, DE, FR, GB, IT, LI, LU, NL, SE)

1. 4-Alkoxy-pyrido[2,3-d]pyrimidin-Derivate der allgemeinen Formel I

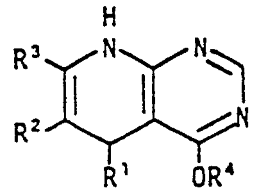

(I)

in welcher

$R^1$ einen unsubstituierten oder einen in 2- oder 3- Position durch Halogen-, Nitro-, Methyl-, Methoxy-, Difluormethoxy-, Trifluormethoxy-, Dimethyl- oder Diethylamino-, Methylthio- oder Trifluormethyl-substituierten oder einen vorzugsweise in 2,3- durch Methoxy- oder Methylendioxy- oder in 2,3- oder 2,6-Position durch Halogenatome, die gleich oder verschieden sein können, disubstituierten Phenylrest,

$R^2$ eine Nitrilgruppe, eine Carboxylgruppe oder einen Alkoxycarbonylrest der allgemeinen Formel II

$$—CO_2R^5 \qquad (II)$$

in welcher $R^5$ einen Methyl-, Ethyl-, n-Propyl-, Isopropyl-, n-Butyl- oder Isobutylrest sowie eine Niederalkoxyalkylgruppe darstellt,

$R^3$ einen Methyl- oder Ethylrest oder eine Aminogruppe,

$R^4$ einen Methyl-, Ethyl-, n-Propyl- oder Isopropylrest bedeutet

sowie gegebenenfalls deren pharmakologisch unbedenkliche Salze.

2. (±)-7-Amino-5,8-dihydro-4-isopropoxy-5-(2-methoxyphenyl)-pyrido-[2,3-d]pyrimidin-6-carbonsäureethylester

3. Verfahren zur Herstellung von 4-Alkoxy-pyrido[2,3-d]-pyrimidin-Derivaten gemäß Anspruch 1 und 2, dadurch gekennzeichnet, daß man 4-Oxo-pyrido[2,3-d]pyrimidin-Derivate der allgemeinen Formel III

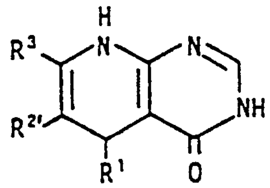

(III)

in welcher $R^1$ und $R^3$ die oben genannte Bedeutung haben und $R^{2'}$ eine Nitrilgruppe oder einen Alkoxycarbonylrest der allgemeinen Formel II bedeutet in an sich bekannter Weise O-alkyliert und die erhaltenen Verbindungen der allgemeinen Formel I gewünschtenfalls anschließend in deren pharmakologisch verträgliche Salze überführt.

4. Verwendung von Verbindungen nach Anspruch 1 und 2 zur Herstellung von Arzneimitteln für die Bekämpfung von Gefäßerkrankungen.

5. Pharmazeutische Zubereitung enthaltend mindestens eine Verbindung nach Anspruch 1 und 2 und pharmazeutisch akzeptable Trägersubstanzen.

**Patentansprüche** (für Vertragsstaat AT)

1. Verfahren zur Herstellung von 4-Alkoxy-pyrido[2,3-d]pyrimidin-Derivaten der allgemeinen Formel I

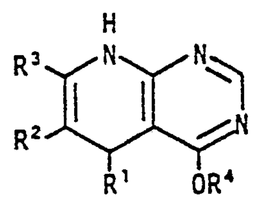

(I)

in welcher

$R^1$ einen unsubstituierten oder einen in 2- oder 3- Position durch Halogen-, Nitro-, Methyl-, Methoxy-, Difluormethoxy-, Trifluormethoxy-, Dimethyl- oder Diethylamino-, Methylthio- oder Trifluormethyl-substituierten oder einen vorzugsweise in 2,3- durch Methoxy- oder Methylendioxy- oder in 2,3- oder 2,6-Position durch Halogenatome, die gleich oder verschieden sein können, disubstituierten Phenylrest,

$R^2$ eine Nitrilgruppe, eine Carboxylgruppe oder einen Alkoxycarbonylrest der allgemeinen Formel II

$$—CO_2R^5 \qquad (II)$$

in welcher $R^5$ einen Methyl-, Ethyl-, n-Propyl-, Isopropyl-, n-Butyl- oder Isobutylrest sowie eine Niederalkoxyalkylgruppe darstellt,

$R^3$ einen Methyl- oder Ethylrest oder eine Aminogruppe,

$R^4$ einen Methyl-, Ethyl-, n-Propyl- oder Isopropylrest bedeutet.

sowie gegebenenfalls von deren pharmakologisch unbedenklichen Salzen, dadurch gekennzeichnet, daß man 4-Oxo-pyrido-[2,3-d]pyrimidin-Derivate der allgemeinen Formel III

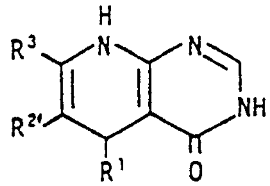

(III)

in welcher $R^1$ und $R^3$ die oben genannte Bedeutung haben und $R^{2'}$ eine Nitrilgruppe oder einen Alkoxycarbonylrest der allgemeinen Formel II bedeutet in an sich bekannter Weise O-alkyliert und die erhaltenen Verbindungen der allgemeinen Formel I gewünschtenfalls anschließend in deren pharmakologisch verträgliche Salze überführt.

2. Verfahren gemäß Anspruch 1 zur Herstellung von (±)-7-Amino-5,8-dihydro-4-isopropoxy-5-(2-methoxyphenyl)-pyrido-[2,3-d]pyrimidin-6-carbonsäureethylester.

3. Verwendung von Verbindungen nach Anspruch 1 und 2 zur Herstellung von Arzneimitteln für die Bekämpfung von Gefäßerkrankungen.

**Claims** (for the Contracting States : (BE, CH, DE, FR, GB, IT, LI, LU, NL, SE)

1. 4-Alkoxy-pyrido [2,3-d] pyrimidine derivatives of the general formula I

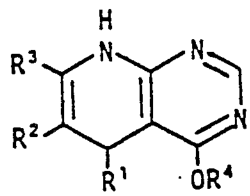

(I)

wherein

$R^1$ represents an unsubstituted or substituted phenyl residue, substituted, in 2 or 3 position, by halogen, nitro, methyl, methoxy, difluoromethoxy, trifluoromethoxy, dimethylamino or diethylamino, methylthio or trifluoromethyl, or a phenyl residue disubstituted, preferably in 2,3 position by methoxy or methylenedioxy, or in 2,3 or 2,6 position by halogen atoms, which may be the same or different,

$R^2$ represents a nitrile group, a carboxyl group or an alkoxycarbonyl residue of the general formula II

$$-CO_2R^5 \quad \text{(II)}$$

in which $R^5$ represents a methyl, ethyl, n-propyl, isopropyl, n-butyl or isobutyl residue as well as a lower alkoxyalkyl group,

$R^3$ represents a methyl or ethyl residue or an amino group,

$R^4$ represents a methyl, ethyl, n-propyl or isopropyl residue,

as well as optionally the pharmacologically acceptable salts thereof.

2. (±)-7-amino-5,8-dihydro-4-isopropoxy-5-(2-methoxyphenyl)-pyrido-[2,3-d]pyrimidine-6-carboxylic acid ethyl ester.

3. Process for the preparation of 4-alkoxy-pyrido [2,3-d] pyrimidine derivatives according to Claim 1 and 2, characterized in that 4-oxo-pyrido [2,3-d] pyrimidine derivatives of the general formula III

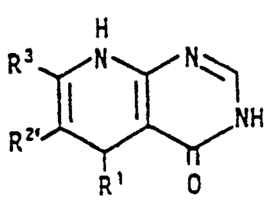

(III)

in which $R^1$ and $R^3$ have the above meaning and $R^{2'}$ represents a nitrile group or an alkoxycarbonyl residue of the general formula II, are O-alkylated in a known manner, and the compounds of the general formula I so obtained, if desired, are subsequently converted into the pharmacologically acceptable salts thereof.

4. Use of compounds according to Claim 1 and 2 for the preparation of medicines in the treatment of vascular diseases.

5. Pharmaceutical preparation containing at least one compound according to claim 1 and 2 and pharmaceutically acceptable carrier substances.

**Claims** (for Contracting State AT)

1. Process for the preparation of 4-Alkoxy-pyrido [2,3-d] pyrimidine derivatives of the general formula I

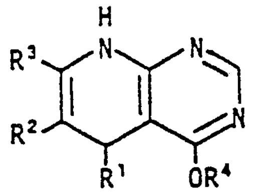

(I)

wherein

$R^1$ represents an unsubstituted or substituted phenyl residue, substituted, in 2 or 3 position, by halogen, nitro, methyl, methoxy, difluoromethoxy, trifluoromethoxy, dimethylamino or diethylamino, methylthio or trifluoromethyl, or a phenyl residue disubstituted, preferably in 2,3 position by methoxy or methylenedioxy, or in 2,3 or 2,6 position by halogen atoms, which may be the same or different,

$R^2$ represents a nitrile group, a carboxyl group or an alkoxycarbonyl residue of the general formula II

$$—CO_2R^5 \qquad (II)$$

in which $R^5$ represents a methyl, ethyl, n-propyl, isopropyl, n-butyl or isobutyl residue as well as a lower alkoxyalkyl group,

$R^3$ represents a methyl or ethyl residue or an amino group,

$R^4$ represents a methyl, ethyl, n-propyl or isopropyl residue,

as well as optionally the pharmacologically acceptable salts thereof, characterized in that 4-oxo-pyrido [2,3-d] pyrimidine derivatives of the general formula III

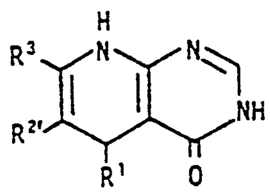

(III)

in which $R^1$ and $R^3$ have the above meaning and $R^{2'}$ represents a nitrile group or an alkoxycarbonyl residue of the general formula II, are O-alkylated in a known manner, and the compounds of the general formula I so obtained, if desired; are subsequently converted into the pharmacologically acceptable salts thereof.

2. Process according to claim 1 for the preparation of (±)-7-amino-5,8-dihydro-4-isopropoxy-5-(2-methoxyphenyl)-pyrido-[2,3-d] pyrimidine-6-carboxylic acid ethyl ester.

3. Use of compounds according to claim 1 and 2 for the preparation of medicines for the treatment of vascular diseases.

**Revendications** (pour les Etats contractants : BE, CH, DE, FR, GB, IT, LI, LU, NL, SE)

1. Dérivés de 4-alkoxy-pyrido-(2,3-d)-pyrimidine de formule générale I :

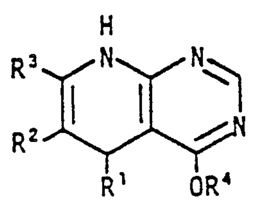

(I)

dans laquelle

$R^1$ est un résidu phényl non substitué ou disubstitué en position 2 ou 3 par un atome d'halogène ou un groupe nitro, méthyl, methoxy, difluorométhoxy, trifluorométhoxy, diméthylamino ou diéthylamino, méthylthio ou trifluorométhyl, de préférence substitué en positions 2 et 3 par des radicaux méthoxy ou

méthylènedioxy ou en positions 2 et 3 ou 2 et 6 par des atomes d'halogène, qui peuvent être identiques ou différents ;

$R^2$ est un groupe nitrile ou carboxyle ou un résidu alkoxycarbonyle de formule générale II ;

$$—CO_2R^5 \quad (II)$$

dans laquelle $R^5$ est un radical méthyl, éthyl, n-propyl, isopropyl, n-butyl ou isobutyl ou un radical alkoxyalkyl inférieur

$R^3$ est un radical méthyl ou éthyl ou un groupe amino ;

$R^4$ est un radical méthyl, éthyl, n-propyl ou isopropyl ;

ainsi que leurs sels pharmacologiquement acceptables.

2. Ester éthylique de l'acide (—)-7-amino-5,8-dihydro-4-isopropoxy-5-(2-méthoxyphényl)-pyrido-[2,3-d]pyrimidine-6-carbonique.

3. Procédé de préparation de dérivés de 4-alkoxy-pyrido-(2,3-d)-pyrimidine selon la revendication 1 ou 2, caractérisé en ce que l'on O-alkyle de façon connue un dérivé 4-oxo-pyrido-[2,3-d]-pyrimidine de formule générale III

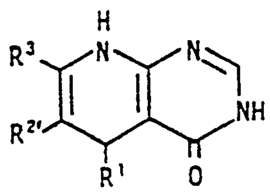

(III)

dans laquelle $R^1$ et $R^3$ ont les significations précitées et $R^{2'}$ est un groupe nitrile ou alkoxycarbonyle de formule générale II puis en ce que l'on transforme éventuellement le composé obtenu, présentant la formule générale I, en un sel pharmaceutiquement acceptable.

4. Application des dérivés selon la revendication 1 ou 2 à la fabrication de médicaments pour le traitement des maladies vasculaires.

5. Composition pharmaceutique comprenant au moins un dérivé selon la revendication 1 ou 2 et un support ou véhicule pharmaceutiquement acceptable.

**Revendications** (pour L'Etat contractant AT)

1. Procédé de préparation de dérivés de 4-alkoxy-pyrido-(2,3-d)-pyrimidine de formule générale I :

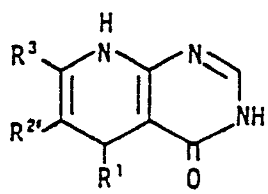

(I)

dans laquelle

$R^1$ est un résidu phényl non substitué ou disubstitué en position 2 ou 3 par un atome d'halogène ou un groupe nitro, méthyl, methoxy, difluorométhoxy, trifluorométhoxy, diméthylamino ou diéthylamino, méthylthio ou trifluorométhyl, de préférence substitué en positions 2 et 3 par des radicaux méthoxy ou méthylènedioxy ou en positions 2 et 3 ou 2 et 6 par des atomes d'halogène, qui peuvent être identiques ou différents ;

$R^2$ est un groupe nitrile ou carboxyle ou un résidu alkoxycarbonyle de formule générale II ;

$$—CO_2R^5 \quad (II)$$

dans laquelle $R^5$ est un radical méthyl, éthyl, n-propyl, isopropyl, n-butyl ou isobutyl ou un radical alkoxyalkyl inférieur ;

$R^3$ est un radical méthyl ou éthyl ou un groupe amino ;

$R^4$ est un radical méthyl, éthyl, n-propyl ou isopropyl ;

ainsi que leurs sels pharmacologiquement acceptables, caractérisé en ce que l'on O-alkyle de façon connue un composé de formule générale III ;

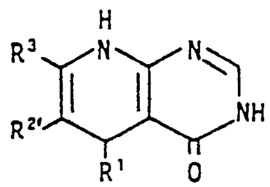

(III)

dans laquelle $R^1$ et $R^3$ ont les significations précitées et $R^{2'}$ est un groupe nitrile ou alkoxycarbonyle de formule générale II puis, en ce que l'on transforme éventuellement le composé obtenu, présentant la formule générale I, en un sel pharmaceutiquement acceptable.

2. Procédé selon la revendication 1, ester éthylique de l'acide(—)-7-amino-5,8-dihydro-4-isopropoxy-5-(2-méthoxyphényl)-pyrido-[2,3-d]pyrimidine-6-carbonique.

3. Application des dérivés selon la revendication 1 ou 2 à la fabrication de médicaments pour le traitement des maladies vasculaires.